# EUROPÄISCHE PATENTSCHRIFT

(11) **EP 2 836 584 B1**
(45) Veröffentlichungstag und Bekanntmachung des Hinweises auf die Patenterteilung: **26.12.2018**
(21) Anmeldenummer: 13709400.9
(22) Anmeldetag: 07.03.2013
(51) Int. Cl.: C12M 1/00, C12N 1/06, C12N 15/10

(54) **VERFAHREN ZUM AUFSCHLIEßEN VON BIOLOGISCHEN ZELLEN**
METHOD FOR DIGESTING BIOLOGICAL CELLS
PROCÉDÉ DE DÉSAGRÉGATION DE CELLULES BIOLOGIQUES

(30) Priorität: 13.04.2012 DE 102012206064
(43) Veröffentlichungstag der Anmeldung: 18.02.2015
(73) Patentinhaber: Robert Bosch GmbH, 70442 Stuttgart (DE)
(72) Erfinder: IBEN, Uwe, 70839 Gerlingen (DE); ROTHACHER, Peter, 76646 Bruchsal (DE); GIEZENDANNER-THOBEN, Robert, 70839 Gerlingen (DE)
(86) Internationale Anmeldenummer: PCT/EP2013/054628
(87) Internationale Veröffentlichungsnummer: WO 2013/152904

(56) Entgegenhaltungen:
- US-A- 4 983 523
- US-A- 6 120 985
- US-A1- 2003 066 915
- US-A1- 2009 016 937
- US-A1- 2010 112 667

## Beschreibung

Die vorliegende Erfindung betrifft ein Verfahren zum Aufschließen von biologischen Zellen sowie eine Vorrichtung zur Durchführung des Verfahrens.

### Stand der Technik

In vielen Bereichen der Biologie und Biochemie und insbesondere auch in der medizinischen Diagnostik und Forschung wird eine Analyse von biologischem Zellmaterial durchgeführt, beispielsweise werden Proteine und andere Zellbestandteile untersucht und charakterisiert. Eine besondere Bedeutung kommt hierbei der Untersuchung des genetischen Materials der Zellen zu. So erfolgt beispielsweise in vielen Fällen ein Nachweis von bestimmten Erregern anhand einer Analyse des genetischen Materials, also insbesondere der DNA. In der herkömmlichen Diagnostik werden Erregerorganismen oftmals anhand einer Kultur des Erregers bestimmt. Nachteilig ist hierbei, dass eine derartige Kultur in der Regel mehrere Tage erfordert und zudem mit einer verhältnismäßig hohen Fehlerwahrscheinlichkeit belastet ist. Andere Nachweismethoden nutzen daher molekularbiologische Methoden. Beispielsweise lässt sich mittels der in den achtziger Jahren entwickelten sogenannten Polymerase-Kettenreaktion (Polymerase Chain Reaction - PCR) erregerspezifische DNA vervielfältigen und nachweisen.

Die Polymerase-Kettenreaktion ist ein hochsensitives In-Vitro-Verfahren zur selektiven Vervielfältigung definierter DNA- oder RNA-Abschnitte. In beispielsweise 20 bis 40 Zyklen wird eine exponentielle Amplifikation, d.h. eine Vervielfältigung der DNA (oder RNA) durchgeführt. Hierbei wird die doppelsträngige DNA zunächst durch Hitze denaturiert. Anschließend erfolgt bei einer niedrigen Temperatur die Anlagerung von spezifischen Oligonukleotiden (Primer) an komplementären Regionen der DNA. Schließlich erfolgt die Synthese der neuen DNA als sogenannte Extension oder Elongation bei einer etwas höheren Temperatur, wobei eine Polymerisation von Desoxyribonukleosidtriphosphaten entlang der DNA-Matrize mit Hilfe des temperaturstabilen Enzyms Taq-DNA-Polymerase stattfindet. Auf diese Weise können hochspezifische Gen-Regionen nachgewiesen werden, um beispielsweise eine bestimmte mikrobielle Familie, Gattung oder Spezies diagnostizieren zu können.

Vor der Durchführung einer Polymerase-Kettenreaktion wird das genetische Material üblicherweise aufgereinigt. In einigen Fällen kann dieses Verfahren auch ohne vorherige Aufreinigung des Zellmaterials bzw. des genetischen Materials durchgeführt werden, wenn die zu untersuchenden Zellen vorab durch eine Behandlung mit erhöhten Temperaturen zerstört und die DNA freigesetzt wurde. Dies ist im Allgemeinen nur bei Gram-negativen Bakterien möglich. Die Zellmembranen von beispielsweise Gram-positiven Bakterien oder Pilzen lassen sich durch eine Hochtemperaturbehandlung im Allgemeinen nicht ausreichend zerstören.

Zum Aufschließen der Zellen bzw. zum Zerstören der Zellmembranen sind daher verschiedene andere Verfahren bekannt. Beispielsweise ist eine enzymatische Lyse der Zellen sehr verbreitet, bei der die Zellmembranen enzymatisch abgebaut werden, beispielsweise durch den Einsatz der Enzyme Proteinase K oder Lysozym. Anschließend sind im Hinblick auf eine Polymerase-Kettenreaktion im Allgemeinen eine Hitzedeaktivierung des eingesetzten Enzyms und eine Abtrennung der Proteine erforderlich, da ansonsten die Aktivität der für die Polymerase-Kettenreaktion eingesetzten Taq-Polymerase eingeschränkt wäre. Eine derartige Reinigung beispielsweise auf einer Silica-Säule erfordert mehrere Wasch- und Elutionsschritte, sodass ein enzymatischer Zellaufschluss verhältnismäßig aufwendig ist. Bei anderen Verfahren wird der Zellaufschluss mittels Ultraschall durchgeführt. Dieses physikalische Verfahren ermöglicht es, eine PCR-Reaktion direkt ohne weitere Aufreinigung der aufgeschlossenen Zellen durchzuführen. Der apparative Aufwand ist allerdings relativ hoch. In dem Fall, dass Vorrichtungen auf Polymerbasis eingesetzt werden, ist zudem eine unkontrollierte Erhitzung des Substrates häufig nicht vermeidbar, sodass Versuchsergebnisse verfälscht werden können. Weiterhin wurde der Einsatz von Lasern für einen Aufschluss von Bakterien beschrieben. Bei dem sogenannten Laser-Kavitationsverfahren wird durch einen Laserpuls Wasser in der Probe so stark erhitzt, dass eine Kavitationsblase entsteht, die letztendlich zur Zerstörung der Organismen in der Probe führt. Hierfür sind Hochenergiepulse im Infrarotbereich erforderlich. Ein anderes Verfahren ist die sogenannte Elektroporation. Durch Hochspannungspulse werden Bakterien porosifiziert und deren Inhaltsstoffe verfügbar gemacht. Insbesondere bei salzhaltigen Lösungen können hierbei Gase entstehen, die beispielsweise zu kleinen Explosionen führen können und schwer handhabbar sind.

Um Analysen mit hohen Probendurchsätzen durchführen zu können, werden sogenannte Biochips eingesetzt, mit welchen verschiedene Analysen, insbesondere auch PCR-Reaktionen, mit sehr kleinen Probenmengen und in automatisierter Weise einschließlich der Probenaufbereitung durchgeführt werden können. Man spricht auch von einem Lab-on-a-Chip-System (LOC). Insbesondere für solche LOC-Systeme ist es vorteilhaft, wenn die Aufarbeitung der Proben und die anschließende Analyse in wenigen Schritten durchgeführt werden können, sodass das Verfahren einer Automatisierung zugänglich ist. Die beschriebenen Verfahren zum Aufschließen der Zellen sind insbesondere im Hinblick auf Gram-positive Bakterien und beispielsweise Pilze verhältnismäßig aufwendig und für ein LOC-System wenig geeignet.

Aus der US-Patentschrift US 6,120,985 geht ein Verfahren zum Aufschließen von Zellen hervor, bei dem die Zellen in gefrorenem Zustand bei einer Temperatur unterhalb des Gefrierpunktes einem erhöhten Druck ausgesetzt werden. Der Druck wird anschließend wieder erniedrigt. Durch einen wiederholten Wechsel dieser Druckverhältnisse erfolgt eine Lyse der Zellen. Die dabei freigesetzten Nukleinsäuren werden anschließend mit einer lonenaustauschermatrix aufgereinigt.

Die US-Patentanmeldungsschrift US 2003/0066915 A1 befasst sich mit einer Vorrichtung zur Zerstörung von Zellen oder Viren. Die Zellen oder Viren befinden sich in einem Behältnis, wobei auf eine vorgespannte Wand dieses Behältnisses mit Hilfe eines Ultraschallgerätes Vibrationen übertragen werden. Diese Vibrationen erzeugen Druckwellen oder Druckpulse in dem Gefäß, die eine Zerstörung der Zellen oder Viren bewirken.

Der vorliegenden Erfindung liegt demgegenüber die Aufgabe zugrunde, ein Verfahren zum Aufschließen von biologischen Zellen bereitzustellen, das mit wenig Aufwand durchführbar ist und das die Übertragung auf ein LOC-System ohne Weiteres erlaubt. Das Verfahren soll dabei allgemein für Bakterien, Pilze, Viren und beispielsweise auch eukaryotische Zellen geeignet sein, wobei unter Verzicht auf Enzyme die Zellmembranen zuverlässig aufgeschlossen werden sollen, sodass das Zellmaterial, beispielsweise die DNA, mit oder ohne weitere Aufreinigung für anschließende Analyseverfahren zur Verfügung steht. Diese Aufgabe wird durch ein Verfahren und eine Vorrichtung zur Durchführung des Verfahrens gelöst, wie es sich aus den unabhängigen Ansprüchen ergibt. Bevorzugte Ausgestaltungen des erfindungsgemäßen Verfahrens bzw. der erfindungsgemäßen Vorrichtung ergeben sich aus den abhängigen Ansprüchen.

### Offenbarung der Erfindung

### Vorteile der Erfindung

Kern der Erfindung ist, dass die biologischen Zellen mit Druckpulsen behandelt werden. Dies führt dazu, dass die Zellhüllen und insbesondere die Zellmembranen zerstört werden und die Zellinhaltsstoffe, beispielsweise die DNA, aus der Zellhülle freigesetzt werden können. Die Druckpulse haben vorzugsweise eine geringe Wellenlänge und eine hohe Amplitude. Die Druckpulse werden mit einem Stoßaktor, der auf dem Wirbelstromprinzip basiert, erzeugt (Wirbelstromaktor). Der Wirbelstromaktor wird insbesondere von einer Spule gebildet, die auf einen elektrischen Leiter wirkt. Der elektrische Leiter ist an oder in dem Reaktionsgefäß mit den aufzuschließenden Zellen angeordnet. Die Zerstörung der Zellmembranen erfolgt also auf rein physikalischem Wege. Es werden keine Enzyme oder sonstigen Zusätze benötigt, die eine weitere Bearbeitung der Probe, beispielsweise eine PCR-Reaktion, stören könnten. Durch den erfindungsgemäßen Einsatz von Druckpulsen kommt es auch nicht zu einer übermäßigen Erwärmung der Probe oder sonstigen unkontrollierbaren Reaktionen, die eine weitere Analyse erschweren könnten. Das erfindungsgemäße Verfahren ist daher mit besonderem Vorteil für eine Automatisierung beispielsweise in Form eines LOC-Systems geeignet. Es können sowohl Gram-negative Bakterien als auch Gram-positive Bakterien, Pilze oder Sporen erfindungsgemäß aufgeschlossen werden. Gegenüber anderen physikalischen Methoden, beispielsweise Ultraschall und Laser, ist der apparative Aufwand für das erfindungsgemäße Verfahren deutlich geringer, sodass das erfindungsgemäße Verfahren wesentlich kostengünstiger durchgeführt werden kann. Durch den Verzicht auf enzymatische und/oder chemische Zusätze ist eine weitere Verarbeitung und/oder Analyse mit wesentlich weniger Aufwand möglich. Zudem werden auch die Kosten für einzusetzende Reagenzien im Vergleich mit herkömmlichen Methoden verringert.

Das erfindungsgemäße Verfahren ist mit besonderem Vorteil für sogenannte µTAS-Systeme (Total Analysis Systems) geeignet, da die erfindungsgemäß aufgeschlossenen Zellen in vielen Fällen ohne weitere Aufreinigung beispielsweise für eine PCR-Reaktion oder andere Verfahren eingesetzt werden können. Auch wenn der jeweilige Anwendungsfall eine weitere Aufreinigung erforderlich macht, ist dies durch den rein physikalischen Aufschluss der Zellen mit wenig Aufwand möglich, da beispielsweise keine Inaktivierung von Enzymen oder Ähnliches erforderlich ist. Das erfindungsgemäße Verfahren ist daher in besonders bevorzugter Weise einer Automatisierung zugänglich.

Die Druckpulse werden durch einen gepulsten Strom in der Spule ausgelöst. Durch die Beaufschlagung der Spule mit einem Strompuls wird ein elektromagnetisches Feld erzeugt. Dieses elektromagnetische Feld induziert im elektrischen Leiter einen Wirbelstrom, dessen elektromagnetisches Feld dem Primärfeld entgegen gerichtet ist. Dadurch kommt es zu einer impulsartigen Abstoßung des elektrischen Leiters. Die Lageänderungen des elektrischen Leiters werden insbesondere über die Wandung des Probengefäßes bzw. Reaktionsbehältnisses auf die Flüssigkeit in dem Behältnis übertragen. Die durch die Bewegung der Wandung induzierten Druckwellen breiten sich in der Flüssigkeit aus und werden an den Behältnisgrenzen reflektiert. Die Druckwellen verursachen Kavitationsblasen und Zugspannungen, die zu einem Zerreißen der Zellhüllen führen, sodass die Zellen aufgeschlossen werden.

Die Behandlung mit Druckpulsen und eine weitere Bearbeitung der Probe, beispielsweise ein sich anschließendes Aufreinigungs- und/oder Analyseverfahren, können in demselben Reaktionsgefäß erfolgen. Dies ist möglich, da das erfindungsgemäße Aufschlussverfahren rein physikalisch ist und in vielen Fällen entsprechende Analysereaktionen, beispielsweise PCR-Reaktionen, ohne weitere Probenaufbereitung möglich sind. Auch wenn eine weitere Aufreinigung oder beispielsweise eine Anreicherung des genetischen Materials erforderlich ist, kann dies mit wenig Aufwand im selben Reaktionsgefäß durchgeführt werden, beispielsweise durch den Einsatz einer geeigneten Filtrationsmatrix. Je nach Anwendungsfall kann es auch bevorzugt sein, den Zellaufschluss und die weitere Probenbearbeitung in separaten Gefäßen bzw. Behältnissen durchzuführen.

Mit besonderem Vorteil werden der erfindungsgemäße Zellaufschluss und eine anschließend gegebenenfalls durchgeführte weitere Probenbearbeitung, beispielsweise ein Aufreinigungs- und/oder Analyseverfahren, in einem Lab-on-a-Chip-System (LOC) durchgeführt. Wie bereits erwähnt, eignet sich das erfindungsgemäße Aufschlussverfahren in besonderer Weise für LOC-Systeme, da der apparative Aufwand verhältnismäßig gering ist und eine gegebenenfalls erforderliche Probenaufbereitung nach dem erfindungsgemäßen Zellaufschluss wenig aufwendig und für eine Automatisierung besonders geeignet ist. Die Verwendung eines LOC für die Durchführung des erfindungsgemäßen Verfahrens zum Aufschließen der Zellen und für die anschließende Bearbeitung und Analyse der Proben kann mit großem Vorteil beispielsweise für automatisierte Diagnoseverfahren beispielsweise zum Nachweis von Krankheitserregern eingesetzt werden.

Vorzugsweise werden die biologischen Zellen zur Vorbereitung des Zellaufschlusses vorab aus einer Flüssigkeit, beispielsweise einer Körperflüssigkeit, abgeschieden und/oder aufgereinigt. Diese Aufreinigung kann beispielsweise mittels eines Filters erfolgen, auf dem die Zellen abgeschieden werden. Ein solcher Filter kann Bestandteil des Reaktionsbehältnisses sein, in dem anschließend der erfindungsgemäße Zellaufschluss stattfindet. Andererseits kann eine Anreicherung und/oder Aufreinigung der Zellen auch vorab in einem separaten Behältnis erfolgen.

Durch die besondere Eignung für eine Automatisierung ist das erfindungsgemäße Verfahren mit besonderem Vorteil in der mikrobiellen Diagnostik einsetzbar. Die Automatisierung des Verfahrens erlaubt insbesondere in Verbindung mit LOC-Systemen eine sehr schnelle und kostengünstige Durchführung mit geringem personellem Aufwand. Das erfindungsgemäße Verfahren kann beispielsweise in medizinischen Laboren, in der Lebensmittelanalytik oder allgemein in der Forschung eingesetzt werden.

Die Erfindung umfasst weiterhin eine Vorrichtung zur Durchführung des beschriebenen Verfahrens, bei dem biologische Zellen durch Behandlung mit Druckpulsen aufgeschlossen werden. Die Vorrichtung umfasst wenigstens ein Reaktionsbehältnis mit einem daran oder darin angeordneten elektrischen Leiter. Weiterhin ist dem Reaktionsbehältnis wenigstens ein Wirbelstromaktor, insbesondere eine Spule, zugeordnet. Eine erfindungsgemäß geeignete Spule umfasst beispielsweise bis zu 100 Wicklungen. Der elektrische Leiter, dem die Spule zugeordnet ist, ist von der Größe her vorzugsweise dem Durchmesser der Spule angepasst. Der elektrische Leiter kann beispielsweise als ringförmige Scheibe, als Platte oder Folie ausgebildet sein. In einer Ausführungsform ist der elektrische Leiter außen auf dem Reaktionsbehältnis angeordnet, beispielsweise aufgeklebt, geschweißt, laminiert oder eingegossen. In einer anderen Ausführungsform kann der elektrische Leiter innerhalb des Reaktionsbehältnisses angeordnet sein, indem er beispielsweise in Form einer Platte oder Scheibe in das Gefäß eingelegt ist. Die Wandung des Reaktionsbehältnisses ist vorzugsweise deutlich flexibler als der elektrische Leiter, also beispielsweise die Platte oder Scheibe, sodass es bei einer Bewegung oder Lageveränderung der Platte oder Scheibe zu einer Verformung der Wandung des Reaktionsbehältnisses kommt. Die Wandung des Reaktionsbehältnisses kann beispielsweise eine verhältnismäßig dünne Kunststofffolie mit einer Dicke von beispielsweise 0,5 bis 1 mm sein, sodass sie wie eine Membran wirkt. Wenn ein Strompuls durch die Spule fließt, beispielsweise durch die Entladung eines Kondensators, wird ein elektromagnetisches Feld erzeugt, dass in dem elektrischen Leiter durch den induzierten Wirbelstrom ein gegengerichtetes Feld erzeugt. Dies führt zu einer Abstoßung und damit Lageveränderung des elektrischen Leiters. Diese pulsartigen Lageveränderungen übertragen sich auf die Behälterwand. Die verhältnismäßig flexible Behälterwand wird dadurch pulsartig verformt. Diese Pulse übertragen sich auf die Flüssigkeit innerhalb des Behältnisses. Es kommt zur Ausbildung von Druckpulsen oder Druckwellen in der Flüssigkeit. Die Druckwellen breiten sich in der Flüssigkeit mit Schallgeschwindigkeit aus und werden an den Innenflächen des Behältnisses reflektiert. Die Druckwellen verursachen Kavitationsblasen und Zugspannungen in der Flüssigkeit, die letztendlich zu einem Zerreißen und Zerstören der biologischen Hüllmembranen der in der Flüssigkeit enthaltenen Organismen bzw. Zellen führen.

Eine Anordnung des elektrischen Leiters außerhalb des Reaktionsgefäßes hat den Vorteil, dass diese Ausführungsform mit wenig Aufwand zu realisieren ist. Zudem verringert diese Ausführungsform die Gefahr von Kontaminationen der Zellprobe. Eine Anordnung des elektrischen Leiters innerhalb des Reaktionsgefäßes hat den Vorteil, dass in der Regel keine Befestigung bzw. Führung des elektrischen Leiters erforderlich ist. Wenn die Geometrie des elektrischen Leiters an die Form der Reaktionsbehältnisses angepasst ist, kann der elektrische Leiter durch Einlegen in das Behältnis positioniert werden. Ein besonderer Vorteil dieser Ausführungsform ist, dass der elektrische Leiter mit Mikrostrukturen versehen sein kann, die bei den durch die elektromagnetisch induzierten Lageveränderungen des elektrischen Leiters innerhalb des Behältnisses Scherkräfte oder Scherspannungen auslösen. Diese Kräfte können die Zerstörung der Hüllschichten der biologischen Zellen unterstützen. Diese Kräfte können in besonders vorteilhafter Weise genutzt werden, wenn die Mikrostrukturen des elektrischen Leiters in gegengleiche Strukturen der Innenwandung des Behältnisses eingreifen.

Der elektrische Leiter kann in einer weiteren Ausführungsform als Stößel oder Kolben realisiert sein. Hierbei kann der gesamte Stößel oder Kolben aus elektrisch leitfähigem Material hergestellt sein. In anderen Ausgestaltungen ist der eigentliche elektrische Leiter in den Stößel oder Kolben integriert. So ist beispielsweise der Stößel oder Kolben selbst aus Kunststoff gefertigt, wobei eine Stirnseite des Stößels oder Kolbens mit einer elektrisch leitfähigen Platte oder Scheibe ausgestattet ist. Die Seite mit der elektrisch leitfähigen Platte oder Scheibe ist dem Wirbelstromaktor zugewandt. Die andere Seite des Stößels oder Kolbens greift am Reaktionsbehältnis an. Ein Vorteil des stößel- oder kolbenförmigen elektrischen Leiters ist, das die Abmessungen des elektrisch leitfähigen Teils des Stößels oder Kolbens größer als das Reaktionsbehältnis sein können, dem der elektrische Leiter zugeordnet ist. Der Wirbelstromaktor, insbesondere die Spule, kann dementsprechend ebenfalls größer ausgelegt sein, sodass stärkere Kräfte induziert werden können. Ein weiterer Vorteil zeigt sich im Vergleich mit der Verwendung einer ringförmigen Scheibe (ohne Stößel oder Kolben). Bei der Verwendung einer Ringscheibe wirken im Bereich der zentralen Aussparung nur wenige Kräfte. Bei dem Einsatz eines Stößels oder eines Kolbens wird die durch die elektromagnetisch induzierte Lageveränderung der integrierten Scheibe oder Platte verursachte Lageveränderung auf die gesamte angreifende Fläche des Stößels oder des Kolbens übertragen, sodass die Kraftübertragung verbessert wird.

Vorzugsweise handelt es sich bei der Vorrichtung zur Durchführung des Verfahrens um ein LOC-System, das wenigstens ein Reaktionskompartiment als Reaktionsbehältnis umfasst. In oder an diesem Reaktionskompartiment ist der elektrische Leiter angeordnet, sodass bei Beaufschlagung des Wirbelstromaktors, insbesondere der Spule, mit Strompulsen durch das induzierte elektromagnetische Feld pulsartige Lageveränderungen des elektrischen Leiters verursacht werden, die in dem Reaktionskompartiment Druckwellen und damit Zugspannungen auslösen. Dies führt zu einer Zerstörung der in dem Reaktionsbehältnis enthaltenen biologischen Zellen.

Die weitere Bearbeitung der Probe und insbesondere die eigentliche Analyse der Zellinhaltsstoffe kann im Prinzip in dem gleichen Kompartiment wie der erfindungsgemäße Zellaufschluss erfolgen. In anderen Ausführungsformen kann hierfür ein separates Kompartiment vorgesehen sein. Für die Durchführung von PCR-Reaktionen sollte das entsprechende Reaktionskompartiment vorteilhafterweise temperierbar sein. Das erfindungsgemäße Verfahren ist jedoch keinesfalls auf eine Kombination mit PCR-Verfahren beschränkt. So kann das erfindungsgemäße Verfahren mit einer Vielzahl verschiedener Methoden zur Detektion verschiedener Zellinhaltsstoffe kombiniert werden, beispielsweise auch in Kombination mit immunologischen Verfahren.

Weiterhin kann die Vorrichtung wenigstens ein weiteres Kompartiment aufweisen, das zur Detektion von Reaktionsprodukten, die beispielsweise bei einer nach dem Zellaufschluss durchgeführten PCR-Reaktion entstehen, und/oder allgemein zur Detektion von Zellinhaltsstoffen vorgesehen ist. Hierfür kann insbesondere ein übliches Array von biologischen Sondenmolekülen vorgesehen sein, die zum Nachweis von bestimmten Molekülen aus der Zelle oder zum Nachweis von beispielsweise PCR-Reaktionsprodukten geeignet sind.

In der erfindungsgemäßen Vorrichtung kann eine Einrichtung zur Aufbereitung des Probenmaterials, beispielsweise Körperflüssigkeiten, vor dem Aufschließen der Zellen vorgesehen sein. Insbesondere kann ein Filter vorgesehen sein, auf dem Zellen aus dem Probenmaterial abgeschieden und angereichert werden, bevor der Zellaufschluss erfolgt. Beispielsweise kann hierfür ein Faserpad aus Glasfasern in den LOC integriert sein. Über dieses Faserpad kann die Probe gepumpt werden, sodass die Zellen auf dem Faserpad abgeschieden werden. Ein anschließender Zellaufschluss kann mit diesen Zellen direkt auf dem Faserpad durchgeführt werden, wobei das Faserpad bzw. der Filter Bestandteil des Reaktionsbehältnisses sein kann, in dem der Zellaufschluss und gegebenenfalls auch eine weitere Bearbeitung der Zellprobe, beispielsweise anschließende Aufreinigungs- und/oder Analyseverfahren, durchgeführt werden.

Weitere Vorteile und Merkmale der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

Kurze Beschreibung der Zeichnungen
- Figur 1: schematische Darstellung eines LOC-Systems zur Durchführung des erfindungsgemäßen Verfahrens in einer ersten Ausgestaltung;
- Figur 2: schematische Darstellung eines LOC-Systems zur Durchführung des erfindungsgemäßen Verfahrens in einer zweiten Ausgestaltung und
- Figur 3: zwei Ausführungsformen einer erfindungsgemäßes Reaktionsgefäßes zum Aufschluss von biologischen Zellen.

### Beschreibung von Ausführungsbeispielen

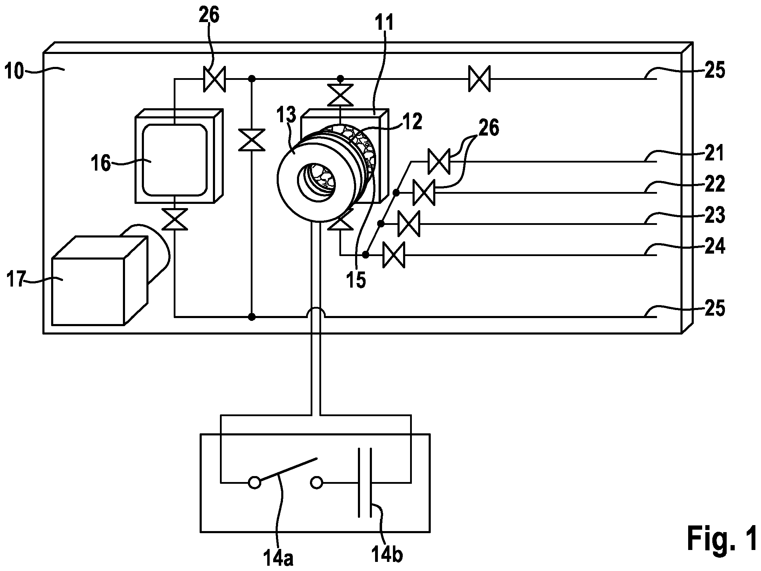

**Figur 1** zeigt in schematischer Weise ein LOC-System, das zur Durchführung des erfindungsgemäßen Zellaufschlusses und zur weitergehenden Analyse der Probe geeignet ist. Bei dieser Vorrichtung handelt es sich beispielsweise um einen spritzgegossenen, polymeren LOC, der in ein entsprechendes Betreibergerät eingesetzt wird, das beispielsweise über einen Mikroprozessor gesteuert wird. Der LOC kann als Einmalartikel vorgesehen sein, da hierdurch insbesondere für Anwendungen in der Medizin Probleme im Zusammenhang mit der Sterilität und mit Kontaminationen vermieden werden. Der LOC 10 umfasst ein Reaktionsbehältnis bzw. Reaktionskompartiment 11. In das Behältnis 11 wird die Probe eingebracht, die die zu untersuchenden Zellen enthält. In dem Behältnis 11 ist ein elektrischer Leiter 12 angeordnet, der in dieser Ausführungsform als eine Lochscheibe aus elektrisch leitendem Material ausgestaltet ist. Der elektrische Leiter sollte eine möglichst gute elektrische Leitfähigkeit aufweisen. Ein geeignetes Material ist beispielsweise Kupfer, aber auch andere Metalle oder Legierungen können verwendet werden. Dem Reaktionskompartiment 11 ist eine Spule 13 als Wirbelstromaktor zugeordnet, die in ihren Abmessungen an die Geometrie des elektrischen Leiters angepasst ist. Die Spule 13 ist über einen schaltbaren elektrischen Kontakt 14a an einen Kondensator 14b angeschlossen. Wenn die Spule 13 mit einem Strompuls beaufschlagt wird, z.B. durch Entladung eines Kondensators, wird hierdurch im elektrischen Leiter 12 ein Wirbelstrom induziert, dessen elektrisches Feld dem Primärfeld entgegen gerichtet ist. Dadurch kommt es zu einer Abstoßung des elektrischen Leiters. Durch die hierdurch verursachte Lageveränderung des elektrischen Leiters wird eine Verformung der Wandung des Reaktionsbehältnisses 11 ausgelöst, die wiederum zur Ausbildung einer Druckwelle führt, die sich mit Schallgeschwindigkeit in der Probenflüssigkeit ausbreitet. Die Druckwelle wird an den inneren Grenzen des Reaktionskompartiments 11 reflektiert. Je nach Druckamplitude werden hierdurch Zugspannungen in der Flüssigkeit erzeugt, die vor allem zu lokalen Kavitationen führen. Die Kavitationsblasen können im Folgenden zusammenfallen und weitere Druckstöße generieren. Die hierdurch verursachten Zugspannungen, Druckwellen und Druckstöße in der Flüssigkeit des Reaktionskompartimentes gegebenenfalls zusammen mit Scherspannungen führen zu einem Zerreißen der Zellhüllen, also insbesondere der Zellmembranen. Die derart erzeugten Druckpulse (Wirbelstromprinzip) zeichnen sich insbesondere durch eine geringe Wellenlänge und eine hohe Amplitude aus. Durch diese Druckpulse werden die Zellhüllen zerstört und das in den Zellen enthaltene Material, also unter anderem das genetische Material, wird freigesetzt und kann für weitere Untersuchungen eingesetzt werden.

Der elektrische Leiter in Form einer Platte, Scheibe oder Folie kann außen auf der Wandung des Reaktionsbehältnisses angeordnet sein. In anderen Ausführungsformen kann die Platte, Scheibe oder Folie innerhalb des Reaktionsbehältnisses 11 vorgesehen sein. Das Reaktionskompartiment bzw. das Reaktionsbehältnis kann beispielsweise mit einer elektrisch leitenden Folie aus Kupfer, Aluminium oder anderen Metallen versehen sein, beispielsweise beklebt, laminiert oder verschweißt. Der Wirbelstromaktor, also insbesondere die Spule 13, wird vorzugsweise in unmittelbarer Nachbarschaft zum elektrischen Leiter angebracht, wobei der Abstand vorzugsweise deutlich kleiner als 1 mm ist, um einen hohen Wirkungsgrad bei den elektromagnetisch induzierten Stößen zu erreichen. Wenn der Wirbelstromaktor mit Strom beaufschlagt wird, erzeugt die Spule ein elektromagnetisches Feld, welches wiederum zu einer Abstoßung des elektrischen Leiters führt. Diese pulsartigen Lageänderungen des elektrischen Leiters werden auf die Wandung des Reaktionsbehältnisses übertragen, wobei es durch die Elastizität der dünnen Wandung zu Verformungen der Wandung kommt, die sich als Druckwellen auf die Flüssigkeit im Behältnis übertragen. Die Wandung des Reaktionsbehältnisses kann beispielsweise aus einem Polymermaterial mit einer Wandstärke von ca. 0,5 bis 1 mm bestehen.

Zum Abscheiden der biologischen Zellen aus der Probenflüssigkeit kann ein Filter 15 in den LOC 10 und insbesondere in das Reaktionskompartiment 11 integriert sein. Mittels des Filters 15 können die Zellen aus der Probenflüssigkeit angereichert werden und für den erfindungsgemäßen Zellaufschluss bereitgestellt werden. Der Filter 15 erlaubt darüber hinaus beispielsweise in vorteilhafter Weise ein Waschen der Zellen.

Der LOC 10 umfasst verschiedene Leitungen oder Kanäle, die für den Fluss der verschiedenen Flüssigkeiten vorgesehen sind, die für die Durchführung des Verfahrens zum Aufschließen der Zellen und für anschließende Aufreinigungs- und/oder Analyseverfahren erforderlich sind. Das Eintragen der Probe kann insbesondere über die Leitung 21 erfolgen. So kann eine zu untersuchende Flüssigkeit, beispielsweise eine Körperflüssigkeit mit zu analysierenden Bakterien bzw. Keimen, über die Leitung 21 in das Reaktionskompartiment 11 gepumpt werden. Als Körperflüssigkeiten kommen beispielsweise Blut, Urin, Sputum, Serum, Plasma, Lymphe, suspendierte Abstriche, broncheoalveoläre Lavagen usw. in Frage. Die flüssige Probe kann zunächst über den Filter 15, der beispielsweise aus Glasfasern besteht, gepumpt werden. Geeignete Glasfasern des Pads können beispielsweise eine Dicke von 0,5 bis 10 µm aufweisen. Hierbei werden die Bakterien oder Keime auf dem Faserpad abgeschieden und können dabei angereichert werden. Anschließend kann beispielsweise mit Puffer (z.B. 2 ml), der über die Leitung 22 zugeführt wird, gewaschen werden. Die durchgelaufenen Flüssigkeiten können insbesondere über die Leitung 25 als Abfall abgeführt werden. Das Fluidmanagement der verschiedenen Flüssigkeiten erfolgt mit Hilfe verschiedener Ventile 26, die in den verschiedenen Leitungen 21 bis 25 vorgesehen sind.

Vor dem eigentlichen Aufschluss der Zellen kann das Reaktionskompartiment 11 bereits beispielsweise mit einem PCR-Mastermix über die Leitung 23 befüllt werden. Ein derartiger PCR-Mastermix enthält insbesondere eine Mischung aus Nukleotiden, Primern, Taq-Polymerase und Puffer. Weiterhin kann in dem Fall, dass ein Filter, wie hier beschrieben, eingesetzt wird, eine Substanz zur Blockierung der Filteroberfläche enthalten sein, beispielsweise BSA, PEG, PPG oder ähnliches. Im Anschluss an den erfolgten Zellaufschluss in der oben beschriebenen Weise kann die PCR-Reaktion unter Verwendung dieser Substanzen durchgeführt werden, wobei weiterhin weitere Substanzen und/oder Puffer über die Leitung 24 zugesetzt werden können. Da die verschiedenen Schritte der PCR-Reaktion bei bestimmten Temperaturen durchgeführt werden müssen, ist die Begrenzung des Reaktionskompartiments 11 temperierbar gestaltet, sodass die Temperatur innerhalb des Reaktionskompartiments entsprechend gesteuert werden und das Reaktionskompartiment den für die PCR-Reaktion üblichen thermischen Zyklen unterworfen werden kann. Als Heizelemente können beispielsweise Peltierelemente, Micro-Hotplates oder konvektive Heiz- und Kühlelemente, gegebenenfalls auch in Kombination, verwendet werden, die von außen an das Reaktionskompartiment angelegt werden können.

Bei den Temperaturzyklen wird die DNA, beispielsweise die erregerspezifische DNA durch Einsatz geeigneter Primer, wie bereits eingangs erläutert, vervielfältigt. Bei Verwendung eines Filterelements 15 kann anschließend die DNA von dem Filterelement 15 eluiert werden. So lassen sich beispielsweise aus 10 ml Urin mit 10⁵ Keimen nach 20 Zyklen 10¹¹ DNA-Moleküle in 50 µl Probenvolumen isolieren. In vergleichbarer Weise lässt sich das erfindungsgemäße Zellaufschlussverfahren beispielsweise auch für eine Amplifikation spezifischer RNA-Abschnitte verwenden.

Nach erfolgter Amplifikation kann die amplifizierte DNA bzw. die Lösung, die diese amplifizierte DNA enthält, in ein weiteres Kompartiment 16 auf dem LOC überführt werden. Hier kann eine Detektion der Reaktionsprodukte erfolgen. Gegebenenfalls erforderliche Hilfsstoffe können zugeführt werden. Die Detektion kann beispielsweise über einen üblichen DNA-Array erfolgen. Weiterhin kann beispielsweise eine elektrophoretische Auftrennung der Reaktionsprodukte mit anschließender Visualisierung vorgesehen sein. Es kann eine Kamera mit Beleuchtung 17 eingesetzt werden. Besonders vorteilhaft ist beispielsweise auch der Einsatz einer sogenannten Realtime-PCR, bei der bereits während der Amplifikation eine Detektion und Analyse der Reaktionsprodukte erfolgt.

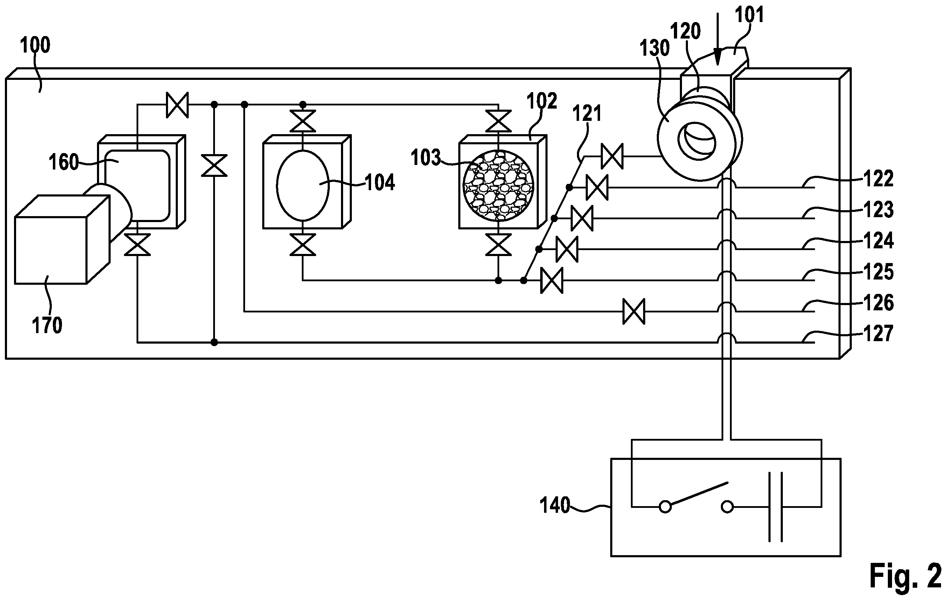

**Figur 2** zeigt eine weitere Ausführungsform eines LOC-Systems 100, das zur Durchführung des erfindungsgemäßen Zellaufschlussverfahrens geeignet ist. In dieser Ausführungsform erfolgen der Zellaufschluss und die weitere Verarbeitung der Probe in voneinander separaten Kompartimenten. Die Probe, also beispielsweise eine Körperflüssigkeit, die den nachzuweisenden Erreger enthält, wird in ein Probenreservoir 101 gegeben. Das Reservoir ist mit einem elektrischen Leiter, insbesondere mit einer Metallschicht 120 ausgestattet. Dem Probenreservoir 101 ist eine elektrische Spule 130 zugeordnet, die mit einer schaltbaren Stromquelle 140 verbunden ist. Die Druckpulse zum Aufschluss der Zellen werden mit Hilfe der elektrischen Spule 130 als Wirbelstromaktor in Verbindung mit dem elektrischen Leiter, also insbesondere der Metallschicht oder Metallscheibe 120, erzeugt. Der elektrische Leiter ist an der Außenseite des Probenreservoirs (Reaktionsbehältnis) angeordnet, beispielsweise aufgeklebt. Durch den Strompuls in der elektrischen Spule 130 wird ein elektromagnetisches Feld erzeugt. Hierdurch wird in dem elektrischen Leiter ein Wirbelstrom induziert, dessen elektrisches Feld dem Primärfeld entgegengesetzt ist, wodurch es zu einer Abstoßung des elektrischen Leiters 120 kommt. Diese pulsartigen Lageveränderungen des elektrischen Leiters 120 übertragen sich auf die elastische Gefäßwandung und erzeugen pulsartige Druckänderungen (Induktionsstöße) in dem Reaktionsbehältnis 101. Die hierdurch letztendlich verursachten Zugspannungen, Druckwellen und Druckstöße in der Flüssigkeit im Probenreservoir 101 führen zu einer Zerstörung der Zellmembranen und damit zu einem Aufschluss der Zellen. Das Probenreservoir 101 als Behältnis, in dem der Zellaufschluss erfolgt, kann, wie hier gezeigt, Teil des LOC-Systems sein. In anderen Ausführungsformen ist es auch möglich, dass dieses Behältnis separat vorgesehen ist und beispielsweise über eine Leitung mit dem LOC für eine weitere Aufreinigung und/oder Verarbeitung der Zellprobe verbunden ist.

Das aufgeschlossene Probenmaterial kann von dem Probenreservoir 101 über eine Leitung 121 in ein weiteres Kompartiment 102 überführt werden, in dem eine Aufreinigung des Probenmaterials durchgeführt werden kann. Beispielsweise kann in dem Kompartiment 102 eine Silica-Matrix 103 integriert sein, über die das genetische Material, insbesondere die DNA, aus der Probe von Verunreinigungen, Zellfragmenten usw. gereinigt wird. Das aufgereinigte genetische Material wird anschließend in ein weiteres Kompartiment 104 überführt. Hier kann eine weitere Reaktion, beispielsweise eine PCR-Reaktion, stattfinden. Die entsprechenden Reagenzien für die Aufreinigung im Kompartiment 102 und für die PCR-Reaktion im Kompartiment 104 werden über die Leitungen 122 (Waschpuffer), 123 (Elutionspuffer), 124 (PCR-Mastermix) und 125 (Hybridisierungspuffer) zugeführt. Nach erfolgter PCR-Reaktion können die PCR-Produkte in ein weiteres Kompartiment 160 überführt werden, in dem die Detektion der Reaktionsprodukte erfolgt. Hierbei kann eine Kamera mit Beleuchtung 170 zum Einsatz kommen, vergleichbar mit der Ausführungsform in Figur 1. Für einen gegebenenfalls erforderlichen Waschpuffer kann eine Leitung 126 und für den Abfall eine Leitung 127 vorgesehen sein. Das Kompartiment 104, das insbesondere für die Durchführung einer PCR-Reaktion vorgesehen ist, ist temperierbar, um die verschiedenen thermischen Zyklen für die Reaktion durchführen zu können.

Der erfindungsgemäße Zellaufschluss ist mit einer Vielzahl von analytischen Methoden kombinierbar. Die hier näher erläuterte PCR-Methode ist nur eines von verschiedenen möglichen Analyseverfahren.

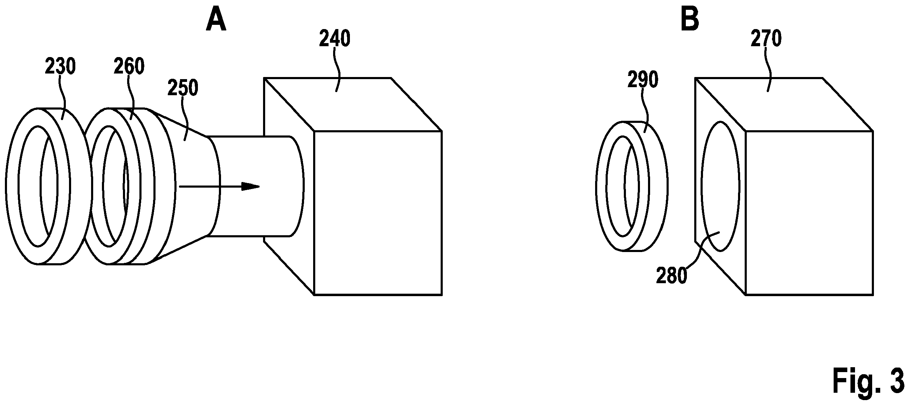

**Figur 3** zeigt weitere Einzelheiten von möglichen Ausführungsformen einer Vorrichtung zur Durchführung des erfindungsgemäßen Zellaufschlusses, wobei eine Gegenüberstellung eines elektrischen Leiters als Stößel (Teilfigur A) und ein elektrischer Leiter als Scheibe (Teilfigur B) gezeigt ist. Die Teilfigur A zeigt den elektrischen Leiter in Form eines Stößels 250, der an der dem Probenreservoir 240 abgewandten Seite mit einer ringscheibenförmigen Metallplatte 260 als eigentlichem elektrischem Leiter versehen ist. Die Metallplatte 260 ist auf den Stößel 250 beispielsweise aufgeklebt. Der Stößel 250 selbst ist beispielsweise als massives spritzgegossenes Kunststoffteil ausgebildet. Der Metallplatte 260 ist eine Spule 230 zugewandt. Die Spule 230 und die Ringscheibe 260 sind in ihrer Größe aneinander angepasst. Das Reservoir 240 enthält eine Suspension mit Zellen, die erfindungsgemäß aufgeschlossen werden sollen.

Durch Beaufschlagung der Spule 230 mit einem Strompuls wird ein elektromagnetisches Feld aufgebaut, wodurch die Metallplatte 260 zusammen mit dem Stößel 250 in Pfeilrichtung abgestoßen wird. Hierdurch werden elektromagnetisch induzierte Stöße auf die elastische Wandung des Probenreservoirs 240 ausgelöst, wodurch es zu einer pulsartigen Druckwellenbildung innerhalb der Flüssigkeit des Probenreservoirs 240 kommt. Diese Druckwellen führen letztendlich zu Zugspannungen in der Probenflüssigkeit im Reservoir 240, die zu einer Zerstörung der Hüllen der Zellen in der Zellsuspension führen. Teilfigur B zeigt ein vergleichbares Reservoir 270, in dem eine Zellsuspension enthalten ist. Das Reservoir 270 ist mit einer außen angeordneten Metallscheibe 280 als elektrischem Leiter ausgestattet. In Verbindung mit einer Strombeaufschlagung der Spule 290 verursacht die Metallscheibe 280 Druckwellen innerhalb der Flüssigkeit, die im Reservoir 270 enthalten ist, wodurch die für eine Zerstörung der Zellhüllen erforderlichen Druckpulse erzeugt werden. Die Spule 290 ist im Vergleich mit der Spule 230 aus der Teilfigur A etwas kleiner, da die Spule in Ihrer Größe an die Größe der Metallscheibe 280 angepasst ist und in etwa den gleichen oder einen etwas kleineren Durchmesser aufweist. Folglich sind die wirkenden Kräfte in der Ausführungsform der Teilfigur B im Vergleich mit der Teilfigur A etwas schwächer.

## Patentansprüche

1. Verfahren zum Aufschließen von biologischen Zellen in einem Reaktionsbehältnis (11; 101; 240; 270), **dadurch gekennzeichnet, dass** die Zellen mit Druckpulsen behandelt werden, wobei die Druckpulse mittels wenigstens eines Wirbelstromaktors (13; 130; 230; 290) in Verbindung mit einem elektrischen Leiter (12; 120; 260; 280), der an oder in dem Reaktionsbehältnis (11; 101; 240; 270) angeordnet ist, erzeugt werden.

2. Verfahren nach Anspruch 1, **dadurch gekennzeichnet, dass** durch eine Beaufschlagung des Wirbelstromaktors (13; 130; 230; 290) mit einem Strompuls ein elektromagnetisches Feld erzeugt wird, wodurch es zu einer Lageveränderung des elektrischen Leiters (12; 120; 260; 280) kommt und ein Druckpuls in dem Reaktionsbehältnis (11; 101; 240; 270) erzeugt wird.

3. Verfahren nach Anspruch 1 oder Anspruch 2, **dadurch gekennzeichnet, dass** die Behandlung mit Druckpulsen und eine weitere Bearbeitung der Zellprobe in demselben Reaktionsbehältnis (11) oder in separaten Reaktionsbehältnissen (101, 102, 104) durchgeführt werden.

4. Verfahren nach einem der vorhergehenden Ansprüche, **dadurch gekennzeichnet, dass** die Behandlung der Zellen mit Druckpulsen und/oder eine weitere Bearbeitung der Zellprobe in einem Lab-on-a-Chip-System (10; 100) durchgeführt werden.

5. Verfahren nach einem der vorhergehenden Ansprüche, **dadurch gekennzeichnet, dass** zur Vorbereitung des Zellaufschlusses die Zellen aus einer Flüssigkeit abgeschieden und/oder aufgereinigt werden, wobei vorzugsweise das Abscheiden der Zellen mittels eines Filters (15) erfolgt.

6. Verfahren nach einem der vorhergehenden Ansprüche, **dadurch gekennzeichnet, dass** das Verfahren für eine mikrobielle Diagnostik eingesetzt wird.

7. Vorrichtung zur Durchführung eines Verfahrens zum Aufschluss von biologischen Zellen durch Behandlung mit Druckpulsen, umfassend wenigstens ein Reaktionsbehältnis (11; 101; 240; 270) mit einem elektrischen Leiter (12; 120; 260; 280) und wenigstens einem dem Reaktionsbehältnis (11; 101; 240; 270) zugeordneten Wirbelstromaktor (13; 130; 230; 290), insbesondere einer Spule, zur Erzeugung eines elektromagnetischen Feldes.

8. Vorrichtung nach Anspruch 7, **dadurch gekennzeichnet, dass** der elektrische Leiter (12; 120; 260; 280) außen an der Wandung des Reaktionsbehältnisses (11; 101; 240; 270) oder in dem Reaktionsbehältnis angeordnet ist.

9. Vorrichtung nach Anspruch 7 oder Anspruch 8, **dadurch gekennzeichnet, dass** die Vorrichtung ein Lab-on-a-Chip-System (10; 100) mit wenigstens einem Reaktionskompartiment als Reaktionsbehältnis (11; 101) ist.

10. Vorrichtung nach einem der Ansprüche 7 bis 9, **dadurch gekennzeichnet, dass** das Reaktionsbehältnis (11) mit dem elektrischen Leiter (12) weiterhin für die weitere Bearbeitung der Zellprobe vorgesehen ist, wobei das Reaktionsbehältnis (11) insbesondere beheizbar ist.

11. Vorrichtung nach einem der Ansprüche 7 bis 9, **dadurch gekennzeichnet, dass** für die weitere Bearbeitung der Zellprobe wenigstens ein weiteres Reaktionskompartiment (104) vorgesehen ist, wobei das weitere Reaktionskompartiment (104) insbesondere beheizbar ist.

12. Vorrichtung nach einem der Ansprüche 8 bis 11, **dadurch gekennzeichnet, dass** das Lab-on-a-Chip-System (10; 100) wenigstens ein weiteres Kompartiment (16; 160) zur Detektion von Reaktionsprodukten und/oder von Zellinhaltsstoffen umfasst.

13. Vorrichtung nach einem der Ansprüche 8 bis 12, **dadurch gekennzeichnet, dass** das Lab-on-a-Chip-System (10; 100) einen Filter (15) zum Abscheiden von Zellen umfasst.

## Claims

1. Method for disrupting biological cells in a reaction container (11; 101; 240; 270), **characterized in that** the cells are treated with pressure pulses, the pressure pulses being generated by means of at least one eddy current actuator (13; 130; 230; 290) in conjunction with an electrical conductor (12; 120; 260; 280) arranged on or in the reaction container (11; 101; 240; 270).

2. Method according to Claim 1, **characterized in that** a current pulse applied to the eddy current actuator (13; 130; 230; 290) generates an electromagnetic field, resulting in a change in position of the electrical conductor (12; 120; 260; 280) and generating a pressure pulse in the reaction container (11; 101; 240; 270).

3. Method according to Claim 1 or Claim 2, **characterized in that** the treatment with pressure pulses and further processing of the cell sample are carried out in the same reaction container (11) or in separate reaction containers (101, 102, 104).

4. Method according to any of the preceding claims, **characterized in that** the treatment of the cells with pressure pulses and/or further processing of the cell sample are carried out in a Lab-on-a-Chip system (10; 100).

5. Method according to any of the preceding claims, **characterized in that** cell disruption preparation involves the cells being segregated and/or purified from a liquid, the cells being preferably segregated by means of a filter (15).

6. Method according to any of the preceding claims, **characterized in that** the method is used for a microbial diagnostics procedure.

7. Device for carrying out a method for disrupting biological cells by means of treatment with pressure pulses, comprising at least one reaction container (11; 101; 240; 270) having an electrical conductor (12; 120; 260; 280) and having at least one eddy current actuator (13; 130; 230; 290), more particularly a coil, assigned to the reaction container (11; 101; 240; 270) for generating an electromagnetic field.

8. Device according to Claim 7, **characterized in that** the electrical conductor (12; 120; 260; 280) is arranged externally on the wall of the reaction container (11; 101; 240; 270) or in the reaction container.

9. Device according to Claim 7 or Claim 8, **characterized in that** the device is a Lab-on-a-Chip system (10; 100) having at least one reaction compartment as reaction container (11; 101).

10. Device according to any of Claims 7 to 9, **characterized in that** the reaction container (11) having the electrical conductor (12) is further intended for the further processing of the cell sample, the reaction container (11) being heatable in particular.

11. Device according to any of Claims 7 to 9, **characterized in that** at least one further reaction compartment (104) is intended for the further processing of the cell sample, the further reaction compartment (104) being heatable in particular.

12. Device according to any of Claims 8 to 11, **characterized in that** the Lab-on-a-Chip system (10; 100) comprises at least one further compartment (16; 160) for detecting reaction products and/or cell components.

13. Device according to any of Claims 8 to 12, **characterized in that** the Lab-on-a-Chip system (10; 100) comprises a filter (15) for segregating cells.)

## Revendications

1. Procédé de désagrégation de cellules biologiques dans un réacteur (11 ; 101 ; 240 ; 270), **caractérisé en ce que** les cellules sont traitées avec des impulsions de pression, les impulsions de pression étant générées au moyen d'au moins un actionneur à courants de Foucault (13 ; 130 ; 230 ; 290) en liaison avec un conducteur électrique (12 ; 120 ; 260 ; 280) qui est disposé sur ou dans le réacteur (11 ; 101 ; 240 ; 270).

2. Procédé selon la revendication 1, **caractérisé en ce qu'**un champ électromagnétique est généré par application d'une impulsion de courant sur l'actionneur à courants de Foucault (13 ; 130 ; 230 ; 290) de manière à modifier la position du conducteur électrique (12 ; 120 ; 260 ; 280) et à générer une impulsion de pression dans le réacteur (11 ; 101 ; 240 ; 270).

3. Procédé selon la revendication 1 ou la revendication 2, **caractérisé en ce que** le traitement avec des impulsions de pression et un traitement ultérieur de l'échantillon de cellules sont effectués dans le même réacteur (11) ou dans des réacteurs séparés (101, 102, 104).

4. Procédé selon l'une des revendications précédentes, **caractérisé en ce que** le traitement des cellules avec des impulsions de pression et/ou un traitement ultérieur de l'échantillon de cellules sont effectués dans un système laboratoire-sur-puce (10 ; 100).

5. Procédé selon l'une des revendications précédentes, **caractérisé en ce que**, pour préparer la désagrégation cellulaire, les cellules sont séparées d'un liquide et/ou purifiées, la séparation des cellules étant de préférence effectuée au moyen d'un filtre (15).

6. Procédé selon l'une des revendications précédentes, **caractérisé en ce que** le procédé est utilisé pour effectuer un diagnostic microbien.

7. Dispositif destiné à mettre en oeuvre un processus de désagrégation de cellules biologiques par traitement avec des impulsions de pression, le dispositif comprenant au moins un réacteur (11 ; 101 ; 240 ; 270) pourvu d'un conducteur électrique (12 ; 120 ; 260 ; 280) et d'au moins un actionneur à courants de Foucault (13 ; 130 ; 230 ; 290) associé au réacteur (11 ; 101 ; 240 ; 270), en particulier une bobine destinée à générer un champ électromagnétique.

8. Dispositif selon la revendication 7, **caractérisé en ce que** le conducteur électrique (12 ; 120 ; 260 ; 280) est disposé à l'extérieur sur la paroi du réacteur (11 ; 101 ; 240 ; 270) ou dans le réacteur.

9. Dispositif selon la revendication 7 ou la revendication 8, **caractérisé en ce que** le dispositif est un système laboratoire-sur-puce (10 ; 100) comprenant au moins un compartiment de réaction servant de réacteur (11 ; 101).

10. Dispositif selon l'une des revendications 7 à 9, **caractérisé en ce que** le réacteur (11) pourvu du conducteur électrique (12) est en outre prévu pour le traitement ultérieur de l'échantillon de cellules, le réacteur (11) pouvant notamment être chauffé.

11. Dispositif selon l'une des revendications 7 à 9, **caractérisé en ce qu'**au moins un autre compartiment de réaction (104) est prévu pour le traitement ultérieur de l'échantillon de cellules, l'autre compartiment de réaction (104) pouvant être chauffé.

12. Dispositif selon l'une des revendications 8 à 11, **caractérisé en ce que** le système laboratoire-sur-puce (10 ; 100) comprend au moins un autre compartiment (16 ; 160) destiné à la détection de produits de réaction et/ou de constituants de cellules.

13. Dispositif selon l'une des revendications 8 à 12, **caractérisé en ce que** le système laboratoire-sur-puce (10 ; 100) comprend un filtre (15) destiné à la séparation de cellules.
